# FASCICULE DE BREVET EUROPEEN

(11) **EP 2 770 925 B1**
(45) Date de publication et mention de la délivrance du brevet: **14.10.2015**
(21) Numéro de dépôt: 12794370.2
(22) Date de dépôt: 25.10.2012
(51) Int. Cl.: A61B 17/70, A61B 17/88

(54) **DISPOSITIF DE MISE EN TENSION D'UNE BANDE SOUPLE ET ENSEMBLE COMPRENANT UN TEL DISPOSITIF AVEC BANDE SOUPLE.**
VORRICHTUNG ZUM SPANNEN EINES FLEXIBLEN BANDES UND ANORDNUNG MIT EINER SOLCHEN VORRICHTUNG MIT EINEM FLEXIBLEN BAND
DEVICE FOR TENSIONING A FLEXIBLE STRIP AND ASSEMBLY COMPRISING SUCH A DEVICE WTIH FLEXIBLE STRIP

(30) Priorité: 28.10.2011 FR 1103319
(43) Date de publication de la demande: 03.09.2014
(73) Titulaire: Implanet, 33650 Martillac (FR)
(72) Inventeur: BACCELLI, Christian, F-33650 Saucats (FR); LE COUEDIC, Regis, F-33000 Bordeaux (FR)
(74) Mandataire: Benech, Frédéric
(86) Numéro de dépôt international: PCT/FR2012/052454
(87) Numéro de publication internationale: WO 2013/060990

(56) Documents cités:
- EP-A1- 1 878 397
- US-A1- 2009 105 715

## Description

La présente invention concerne un dispositif de mise en tension d'une bande souple pour maintien d'un élément osseux sur un implant, comportant une tige ayant une première extrémité munie de moyens d'appui sur une barre longitudinal de fixation de l'implant, une pièce mobile d'accrochage de la bande souple sur une portion du dispositif et des moyens de blocage réglable de la pièce mobile en translation par rapport à cette portion.

Elle concerne également un ensemble comprenant un tel dispositif et la bande souple qui va avec.

Elle trouve une application particulièrement importante bien que non exclusive dans le domaine du redressement de la colonne vertébrale d'un patient présentant une courbure anormale.

Dans ce cas, les vertèbres n'étant pas alignées correctement les unes par rapport aux autres par rapport à l'axe vertébral, elles présentent des inclinaisons entre elles.

Afin de redresser l'ensemble, il est connu de remettre à une distance sensiblement équivalente des bords latéraux des vertèbres de part et d'autre de la colonne vertébrale, par le biais de barres longitudinales reliant entre elle, soit des vis, que l'on insère dans les vertèbres elles-mêmes, soit des crochets que l'on introduit le long du canal rachidien.

De tels dispositifs présentent cependant des inconvénients car ils sont agressifs.

Pour palier à ces inconvénients, il a été proposé un lien flexible de fixation de la vertèbre sur la pièce de: liaison elle-même fixé à la barre longitudinale permettant le redressement.

Des moyens de blocage du lien flexible par refermeture de la pièce de liaison sur la barre sont alors nécessaires.

Le problème que cherche à résoudre l'invention est ici celui de la mise en tension de ce lien flexible de fixation de la vertèbre sur la pièce de liaison.

En effet, il est important que l'utilisateur lorsqu'il va mettre en place cette liaison puisse resserrer progressivement cette dernière sans pour autant endommager l'élément osseux qui vient donc être comprimé par la bande souple.

Il existe actuellement des ancillaires de mise en tension munis d'une tige, d'une pièce mobile en translation le long de la tige et d'un élément monté sur la pièce mobile tendant horizontalement à écarter l'extrémité de la tige par rapport à la pièce.

Un tel ancillaire présente cependant des inconvénients.

Il nécessite en effet de tirer dans la prolongation du lien lui-même qui doit être vrillé pour pouvoir permettre sa mise en tension.

De plus aucun retour manuel de la tension n'est possible avec un tel instrument qui nécessite du coup pour éviter de broyer l'élément osseux un système dynamométrique réglable permettant d'arrêter la mise en tension au delà d'une certaine valeur déterminée.

La présente invention vise à fournir un dispositif de mise en tension d'une bande souple répondant mieux que ceux antérieurement connus aux exigences de la pratique, notamment en ce qu'elle va permettre une plus grande flexibilité, une meilleure sensibilité au niveau de la mise en tension du fait du concept même des outils utilisés pour une telle mise en tension, et en ce qu'elle présente des possibilités de réglage très améliorées et ce de façon simple.

L'invention permet en outre de compenser les résistances générées par les défauts de glissement de la bande souple autour de l'attache anatomique.

Dans ce but l'invention propose notamment un dispositif de mise en tension d'une bande souple pour maintien d'un élément osseux sur un implant, comportant une tige ayant une première extrémité munie de moyens d'appui sur une barre longitudinale de fixation de l'implant, une pièce mobile d'accrochage de la bande souple sur une portion du dispositif et des moyens de blocage réglable de la pièce mobile en translation par rapport à ladite portion du dispositif, caractérisé en ce que la tige comprend un manche de préhension à sa deuxième extrémité, en ce que ladite portion du dispositif forme un angle avec la tige à laquelle elle est fixée rigidement à un niveau intermédiaire avec renvoi d'angle de la bande et en ce que, ladite bande souple comprenant deux bouts libres, ladite pièce mobile comprend une roue libre en rotation par rapport à un axe perpendiculaire à ladite portion, de guidage de la bande autour de son pourtour arrondi comportant deux éléments distincts d'ancrage de la bande souple, à savoir un premier élément pour un desdits bouts de la bande souple et un deuxième élément pour l'autre bout.

Les moyens de vissage manuel d'actionnement de la pièce mobile elle-même déportée sur une branche latérale formant un angle, permettent une mise en tension progressive et contrôlée de la bande souple.

Avantageusement les moyens d'appui sont de part et d'autre de l'implant par rapport à la tige.

Dans des modes de réalisation avantageux, on a de plus recours à l'une et/ou à l'autre des dispositions suivantes :
- les deux éléments d'ancrage sont sensiblement diamétralement opposés;
- la roue est en forme de disque circulaire ou ovale tronqué comportant une gorge de guidage de la bande sur son pourtour;
- la bande comportant d'un côté un embout d'extrémité en lamelle souple et de l'autre coté un pion d'accrochage, le premier élément est formé par une saignée oblique dans le plan de la roue de rétention de la lamelle, et le deuxième élément par un crochet d'accrochage du pion;
- les moyens de blocage réglable comprennent des moyens de vissage propres à actionner la pièce mobile;
- les moyens de blocage réglables comportent une crémaillère solidaire de la portion de dispositif, et les moyens de vissage comprennent un système à cliquet avec anti-retour et clé d'actionnement solidaire de la pièce mobile;.
- le système à cliquet comporte un bras de levier de relâche du cliquet;
- les moyens de vissage comportent au moins une vis papillon;
- le dispositif comporte une pièce de liaison entre la tige et la portion de dispositif formée par un bloc muni de deux pignons d'axes parallèles, entre eux de renvoi d'angle de la dite bande dans son plan, ledit bloc étant solidaire de ladite tige;
- la pièce de liaison est mobile le long de la tige;
- l'angle de ladite portion de dispositif par rapport à la tige est réglable;
- l'angle de ladite portion de dispositif par rapport à la tige est compris entre 90° et 130° ;
- l'angle de ladite portion de dispositif par rapport à la tige est de 90° ;

L'invention propose également un ensemble comprenant un dispositif tel que précédemment décrit et une bande souple.

Elle concerne également un procédé de mise en tension d'une bande sur un os et/ou une vertèbre utilisant un ensemble tel que décrit ci-dessus.

L'invention sera mieux comprise à la lecture de la description qui suit d'un mode de réalisation donné ci-après à titre d'exemple non limitatif.

La description se réfère aux dessins qui l'accompagnent dans lesquels :
La figure 1 est une vue en perspective d'un corps de fixation sur une barre, mettant en oeuvre une bande souple propre à être tendue avec le dispositif selon l'invention (pour maintien d'un élément osseux).
La figure 2 est une vue de dessous d'une vertèbre montrant le corps de fixation sur la barre et la bande souple sectionnée après serrage de la bande par le dispositif selon l'invention.
La figure 3 est une vue en perspective axionométrique du dispositif selon un mode de réalisation de l'invention.
La figure 4 est une vue latérale d'en ensemble comprenant le dispositif de la figure 3 et une bande souple.
La figure 5 est une vue en perspective partielle de la pièce mobile du dispositif de la figure 3.
La figure 6 est une vue en perspective d'une bande souple de liaison selon un mode de réalisation de l'invention.

Dans la suite de la description on utilisera les mêmes numéros de référence pour désigner les mêmes éléments.

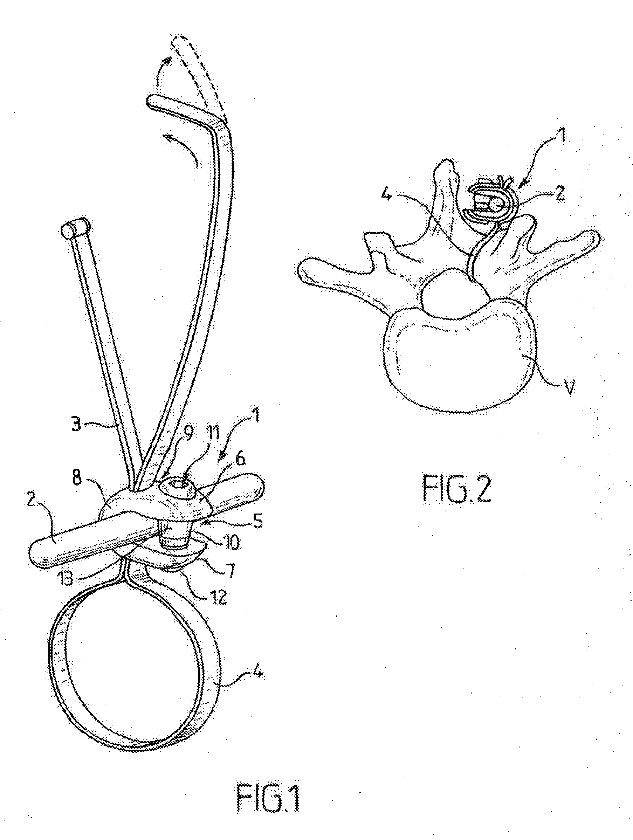

La figure 1 montre un corps 1 de fixation sur une barre cylindrique 2 et une bande souple 3 en polymère tressé, par exemple en polyester de 6 mm de large et de 30 cm de long pour former la boucle 4.

Des moyens de blocage 5 réglables de la bande souple 3. sur le corps 1 de fixation sont prévus. Le corps 1 de fixation est par exemple formé par une pièce d'un seul tenant formant une prince présentant une section transversale en forme de U, ledit U comprenant deux branches épaisses 6, 7 d'une section transversale sensiblement en forme de demi-ovale, symétriques par rapport à un plan longitudinal et reliées entre elles par une partie de liaison 8 en forme de demi-anneau torique formant d'un côté le fond en demi-cylindre du U et de l'autre côté les parois externes arrondies des branches 6 et 7.

La paroi de fond du U est de forme complémentaire à celle de la barre 2 ou sensiblement complémentaire.

Chaque branche 6, 7 comporte un évidement 9, par exemple en forme de fente large, par exemple cinq à dix fois plus large que l'épaisseur de la tresse pour faciliter son introduction lors de l'opération.

Chaque branche 6 et 7 comporte un orifice cylindrique de passage des moyens 5 de blocage, à savoir un alésage de diamètre D et un orifice cylindrique taraudé de vissage du diamètre d < D.

Les moyens 5 de blocage sont formés par une pièce de liaison 10, ou vis, munie d'un côté d'une tête 11 de passage dans l'alésage du U et de l'autre d'une extrémité 12 de vissage dans ledit orifice cylindrique taraudé en vis à vis.

Dans le mode de réalisation plus particulièrement décrit ici, la tête de la vis 11 comprend une partie supérieure cylindrique et une partie inférieure 13 tronconique vers le bas, agencée pour comprimer la barre 2 au fur et à mesure du vissage de la pièce.

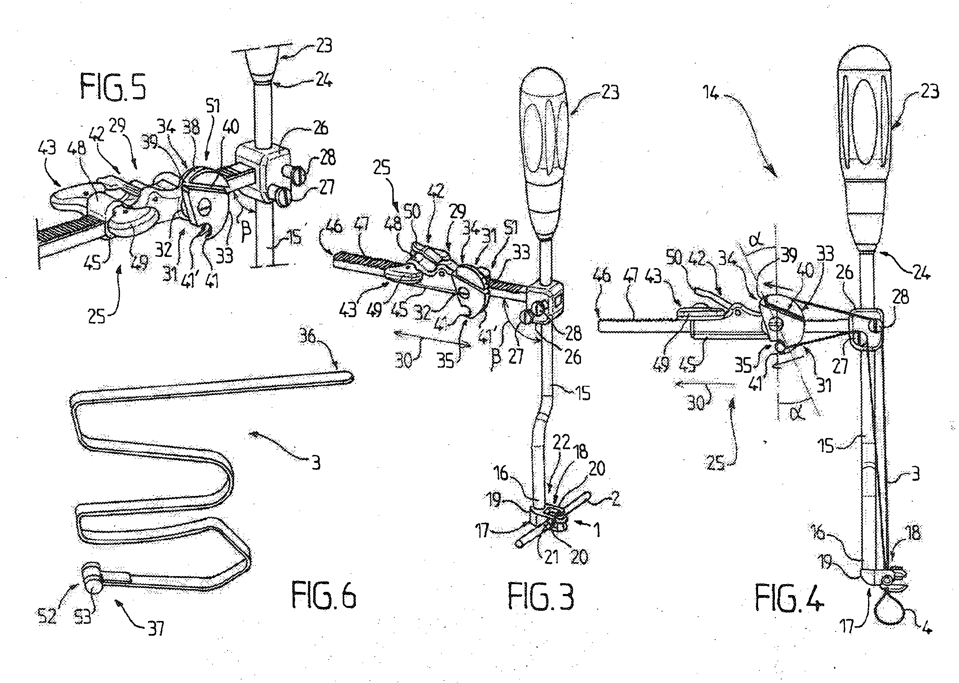

On a représenté sur la figure 2 la vertèbre V telle que fixée par l'intermédiaire de la boucle 4 à la barre 2 via le corps 1 pour permettre la mise en tension de la boucle 4 sur l'os V par un dispositif i4 comme représenté sur les figures 3, 4 et 5.

Le dispositif 14 de mise en tension de la bande souple 3 par l'intermédiaire de la boucle 4, comporte une tigre 15 longiligne par exemple de 40 cm de long et cylindrique muni à une première extrémité 16 de moyens d'appui 17 par exemple constitués par une pièce solidaire de l'extrémité 16 présentant une partie 18 en crochets cylindriques, de forme complémentaire et agencée pour coopérer avec la barre 2.

Plus précisément la pièce 17 est par exemple formée par une partie de talon 19 solidaire de l'extrémité 16 prolongée de chaque côté par deux branches symétriques parallèles 20 munies à leur extrémité de deux parties d'épaulement 21 agencées pour coopérer avec la barre 2.

Les deux branches 20 sont elles-mêmes séparées par une ouverture 22 dans laquelle vient s'insérer le corps 1 de fixation sur la barre de façon connue en elle-même.

Le dispositif comporte un manche 23 de préhension manuelle dans le prolongement de la tige 15 à sa deuxième extrémité 24.

Le dispositif selon le mode de réalisation de l'invention plus particulièrement décrit ici comporte également une portion .25 formant un angle β avec la tige 15 à laquelle elle est fixée rigidement à un niveau intermédiaire par l'intermédiaire d'une pièce de liaison 26 entre tige et dite portion, formée par un bloc par exemple sensiblement parallélépipédique muni de deux pignons 27 et 28 d'axe parallèle entre eux, perpendiculaires à l'axe de la tige 15, de renvoi d'angle de la bande 3 dans son plan, vers la portion de tige 25.

Il est prévu une pièce 29 mobile en translation (flèche 30) par rapport à la portion 25.

La pièce 29 mobile comprend en outre une roue 31 de compensation montée libre en rotation autour d'un axe 32 qui les relie, l'axe 32 étant perpendiculaire au déplacement de la pièce 29 mobile et positionnant en vis-à-vis la tranche 33 de la roue 31 de compensation et les deux pignons 27, 28.

La roue 31 de compensation est de forme générale en disque circulaire ou ovoïde, tronqué, et comprend des moyens 34, 35 d'ancrage des extrémités 36, 37 de la bande souple 3 (voir figure 6).

Elle comprend sur son pourtour arrondi une gorge 38 de guidage de la bande souple 3 de largeur légèrement supérieure à ladite bande souple 3.

Les moyens d'ancrage 34, 35 qui permettent de solidariser les extrémités 36, 37 de la bande souple 3 à la roue 31 de compensation sont sensiblement diamétralement opposés.

En situation de repos, c'est-à-dire lors qu'aucun effort de tension n'est appliqué à la roue 31 de compensation, celle-ci est équilibrée de manière à ce que les moyens d'ancrage 34, 35 soient alignés verticalement et approximativement parallèles de l'axe de la tige 15.

Le premier moyen d'ancrage 34 supérieur comprend à l'extrémité de la gorge 38 de guidage un retour 39 formé par une saignée 40 dans la roue 31 de compensation.

La saignée 40 est d'épaisseur adaptée pour accueillir et retenir la bande souple 3 et traverse la surface de ladite roue 31 de compensation.

Le retour équilibre ainsi les efforts de tractions de la bande 3 et assure alors un maintien optimum de ladite bande 3.

Le second moyen d'ancrage 35 comprend deux dents 41, 41' courbées adaptées à collaborer avec un cylindre et à maintenir celui-ci en place.

Les deux dents 41, 41' sont espacées au moins de la largeur de la bande 3.

La pièce mobile 29 comprend en outre des moyens 42 de blocage réglables de la pièce mobile 29 dans sa dite translation, munis de moyens 43 de vissage.

En référence plus précisément à la figure 5 les moyens 42 de blocage réglable comportent une crémaillère solidaire de la portion 25, les moyens de vissage 43 comprenant quant à eux un système à cliquet avec anti-retour et clé d'actionnement solidaire de la pièce mobile 29.

Les moyens 42 de blocage réglables comportent un corps 45 percé d'un alésage au travers du quel passe l'extrémité 46 de la portion 25 qui est fixée à la tige 15 par la pièce de liaison 26.

L'extrémité 46 qui comporte sur sa partie supérieure les dents 47 de la crémaillère va coopérer à flottement doux avec l'alésage de la pièce 45 qui comporte dans sa partie supérieure un ergot 48 percé d'un alésage latéral au travers duquel passe l'axe des papillons 49 d'actionnement manuel d'une roue dentée agencée pour coopérer avec les dents 47 de la crémaillère de sorte que lorsque l'on va tourner les vis papillons la pièce mobile 29 va se déplacer transversalement le long de la portion 25.

Des moyens connus en eux-mêmes à cliquet sont prévus actionnables et déverrouillables lorsqu'on lève le levier 50 anti-retour de façon connue en elle-même.

Ceci permet en effet de relâcher le cliquet et de libérer la tension de la bande souple.

La bande souple 3, plus exactement les extrémités 36, 37 de cette bande souple 3 sont solidaires de la roue de compensation 31 fixé à l'extrémité 51 de la pièce mobile 29, de sorte que lorsqu'on déplace la pièce mobile 29 vers l'extérieur de la tige 15, la bande 3 et la boucle 4 se retrouvent mises en tension.

L'angle β entre la portion 25 et la tige 15 est par exemple réglable pour être bloqué de façon connue en elle-même.

La bande souple 3 va maintenant être décrite en référence à la figure 6.

Elle est de forme globalement parallélépipédique, une des extrémités 36 de la bande 3 souple pouvant être de forme biseautée, en pointe ou arrondie.

Cette même extrémité 36 peut être renforcée par une lamelle métallique de même forme que l'extrémité de la bande souple 3, à qui il peut être donné une forme de crochet malléable permettant l'introduction et le passage sous la vertèbre.

A l'autre extrémité 37 de la bande se trouve un moyen d'ancrage.

Dans le mode de réalisation plus particulièrement décrit ici, le moyen d'ancrage est constitué d'un pion d'accrochage 52.

Le pion d'accrochage 52 est formé par un cylindre 53 orthogonal à la bande 3 souple comprenant sur sa partie médiane une gorge de largueur légèrement supérieure à la bande 3 souple et autour de laquelle une partie de ladite bande 3 s'enroule de manière à former prise dudit cylindre 53.

On va maintenant décrire le fonctionnement d'un dispositif selon l'invention.

L'utilisateur va tout d'abord former la boucle 4 autour de l'élément osseux.

Pour cela il fait passer une première fois la première extrémité 36 de la bande 3 souple dans le corps 6 de fixation et fait le tour de la partie osseuse et repasse par le corps 6 de fixation formant ainsi une boucle 4.

Le profilé de l'extrémité de la bande souple 3 ainsi que son renfort métallique étant de dimensions faibles et d'une certaine rigidité, cette opération en est facilitée.

Puis l'utilisateur fixe dans la saignée 40 de la roue de compensation 31 la première extrémité 36 au premier moyen 34 d'ancrage de la roue 31 de compensation en faisant reposer la bande 3 souple sur la gorge 38 de guidage de la roue 31 de compensation.

Une seule et même extrémité passe par le corps de fixation 32.

La boucle 4 autour de l'os est alors simple, sans noeud ce qui implique un gain de temps de pose pour le chirurgien et permet une plus grande sécurité pour cette opération délicate et complexe dans un milieu tissulaire.

Il fixe alors la seconde extrémité 37 de la bande 3 souple au second moyen 35 d'ancrage en insérant le pion d' ancrage 52 de l'extrémité dans les dents 41, 41' du moyen 35 d'ancrage en laissant passer la bande 3 au travers de l'espace entre les deux dents 41, 41' et reposant sur le pion 27.

L'utilisateur n'est donc pas astreint de fixer l'extrémité de la bande 3 souple pour former une courroie de quelque manière que ce soit.

Le pion d'ancrage 52 de la bande 3 souple est retenu par la forme incurvée des dents 41, 41' et par un léger clipsage du cylindre 53 dans celles-ci.

L'équilibrage de la roue 31 de compensation permet alors de ne pas induire d'effort parasite sur la tension de la bande 3 souple et donc de la partie osseuse.

Le dimensionnement de la bande 3 souple aura été au préalable calculé de façon à ce que la bande 3 soit à peu près en position tendue. Au cas où celle-ci serait plus important, il est possible aussi de bouger par exemple dans un mode de réalisation la pièce 26 de fixation de la portion 25 sur ladite tige 15 de façon à obtenir la tension initiale recherchée.

La tension s'effectue ensuite en tournant les papillons 49, ce qui entraîne la roue dentée qui engraine sur la crémaillère.

Lorsque la brande 3 souple glisse sans résistance sur l'os, la roue 31 de compensation agit comme une fixation rigide des extrémités de la bande 3 souple et reste dans sa position naturelle d'équilibrage.

Lorsque le glissement de la bande 3 souple est freiné dans son glissement, le brin correspondant subit une surtension par rapport à l'autre brin qui déséquilibre la roue 31 de compensation qui s'incline d'un angle α pour compenser graduellement cette résistance.

Comme il va de soi et comme il résulte également de ce qui précède, la présente invention n'est pas limitée aux modes de réalisation plus particulièrement décrits. Elle en embrasse au contraire toutes les variantes et notamment celles où il n'y a qu'une seule clé papillon, celle où les moyens de vissage sont différents et/ou les moyens d'ancrage 34, 35 de la bande souple 3 sont constitués différemment.

## Revendications

1. Dispositif de mise en tension d'une bande souple. (3) pour maintien d'un élément osseux sur un implant, comportant une tige (15) ayant une première extrémité (16) munie de moyens (17) d'appui sur une barre longitudinale de fixation de l'implant, une pièce (29) mobile d'accrochage de la bande souple (3) sur une portion (25) du dispositif et des moyens de blocage (42) réglable de la pièce mobile (29) en translation par rapport à ladite portion (25) du dispositif,
**caractérisé en ce que** à tige (15) comprend un manche (23) de préhension à sa deuxième extrémité (24), **en ce que** ladite portion (25) du dispositif (14) forme une angle avec la tige (15) à laquelle elle est fixée rigidement à un niveau intermédiaire avec renvoi d'angle (27, 28) de la bande (3) et **en ce que**, ladite bande souple comprenant deux bouts (36, 37) libres, ladite pièce mobile (29) comprend une roue libre (31) en rotation par rapport à on axe (32) perpendiculaire à ladite portion (25), de guidage de la bande (3) autour de son pourtour arrondi (33) comportant deux éléments (34, 35) distincts d'ancrage de la bande souple (3), à savoir un premier élément (34) pour un desdits bouts de la bande souple (3) et un deuxième élément (35) pour l'autre bout.

2. Dispositif selon la revendication 1, **caractérisé en ce que** les deux éléments (34, 35) d'ancrage sont sensiblement diamétralement opposés.

3. Dispositif selon la revendication 2, **caractérisé en ce que** la roue (31) est en forme de disque circulaire on ovale tronqué comportant une gorge (38) de guidage de la bande (3) sur son pourtour (33).

4. Dispositif selon l'une quelconque des revendications précédentes, **caractérisé en ce que**, la bande (3) comportant d'un côté (36) un embout d'extrémité en lamelle souple et de l'autre côté (37) un pion d'accrochage (52), le premier élément (34) est formé par une saignée (40) oblique dans le plan de la roue (31) de rétention de la lamelle, et le deuxième élément (35) par un crochet (41, 41') d'accrochage du pion (52).

5. Dispositif selon l'une quelconque des revendications précédentes, **caractérisé en ce que** les moyens de blocage (42) réglable comprennent des moyens de vissage (43) propres à actionner la pièce mobile (29).

6. Dispositif selon la revendication 5, **caractérisé en ce que** les moyens de blocage (42) réglables comportent une crémaillère solidaire de la portion (25) de dispositif, et les moyens de vissage (43) comprennent un système à cliquet avec anti-retour et clé d'actionnement solidaire de la pièce mobile (29).

7. Dispositif selon la revendication 6, **caractérisé en ce que** le système à cliquet comporte un bras de levier (50) de relâche du cliquet.

8. Dispositif selon l'une quelconque des revendications 5 à 7, **caractérisé en ce que** les moyens de vissage (43) comportent au moins une vis papillon (49).

9. Dispositif selon l'une quelconque des revendications précédentes, **caractérisé en ce que** il comporte une pièce de liaison (26) entre la tige (15) et la portion (25) de dispositif formée par un bloc muni de peux pignons (27, 28) d'axes parallèles entre eux de renvoi d'angle de la dite bande (3) dans son plan, ledit bloc étant solidaire de ladite tige (15).

10. Dispositif selon la revendication 9, **caractérisé en ce que** la pièce de liaison (26) est mobile le long de la tige (15).

11. Dispositif selon l'une quelconque des revendications précédentes, **caractérisé en ce que** l'angle de ladite portion (25) de dispositif par rapport à la tige (15) est réglable.

12. Dispositif selon l'une quelconque des revendications précédentes, **caractérisé en ce que** l'angle de ladite portion (25) de dispositif par rapport à la tige (15) est compris entre 90° et 130°.

13. Dispositif selon la revandication 12, **caractérisé en ce que** l'angle de ladite portion (25) de dispositif par rapport à la tige (15) est de 90°.

14. Ensemble **caractérisé en ce qu'**il comprend un dispositif (14) selon l'une quelconque des revendications précédentes et une bande souple (3).

## Patentansprüche

1. Vorrichtung zum Spannen eines flexiblen Bandes (3) zum Haltern eines Knochenelements an einem Implantat, umfassend einen Stift (15) mit einem ersten Ende (16) mit Mitteln (17) zum Abstützen auf einen Längsstab zur Befestigung des Implantats, ein bewegliches Teil (29) zum Einhängen des flexiblen Bandes (3) an einem Abschnitt (25) der Vorrichtung und einstellbare Mittel (42) zur Arretierung des beweglichen, in Bezug auf den Abschnitt (25) der Vorrichtung verschieblichen Teils (29),
**dadurch gekennzeichnet,**
**dass** der Stift (15) an seinem zweiten Ende (24) einen Greifstiel (23) aufweist, dass der Abschnitt (25) der Vorrichtung (14) einen Winkel mit dem Stift (15) bildet, an dem er auf einer Zwischenebene mit Winkelgetriebe (27, 28); für das Band (3) starr befestigt ist, und dass, wobei das flexible Band zwei freie Endstücke (36, 37) aufweist, das bewegliche Teil (29) ein in Bezug auf eine zum Abschnitt (25) senkrechte Achse (32) drehendes Freilaufrad (31) für die Führung des Bandes (3) um seinen gerundeten Umfang (33) mit zwei getrennten Elementen (34, 35) für die Verankerung des flexiblen Bandes (3) umfasst, und zwar einem ersten Element (34) für eines der Endstücke des flexiblen Bandes (3) und einem zweiten Element (35) für das andere Endstück.

2. Vorrichtung nach Anspruch 1,
**dadurch gekennzeichnet,**
**dass** die zwei Verankerungselemente (34, 35) im Wesentlichen diametral entgegengesetzt sind.

3. Vorrichtung nach Anspruch 2, **dadurch gekennzeichnet, dass** das Rad (31) die Form einer kreisförmigen oder ovalen abgeschnittenen Scheibe aufweist mit einer Nut (38) für die Führung des Bandes (3) auf ihrem Umfang (33).

4. Vorrichtung nach einem der vorhergehenden Ansprüche,
**dadurch gekennzeichnet,**
**dass**, vorausgeschickt, dass das Band (3) auf der einen Seite (36) ein Endstück aus einer flexiblen Lamelle und auf der anderen Seite (37) einen Einhängzapfen (52) aufweist, das erste Element (34) durch eine schräge Aussparung (40) in der Ebene des Rads (31) für das Zurückhalten der Lamelle gebildet ist und das zweite Element (35) durch einen Haken (41, 41') für das Einhängen des Zapfens (52) gebildet ist.

5. Vorrichtung nach einem der vorhergehenden Ansprüche,
**dadurch gekennzeichnet,**
**dass** die einstellbaren Arretierungsmittel Verschraubungsmittel (43) umfassen, die geeignet sind, das bewegliche Teil (29) zu betätigen.

6. Vorrichtung nach Anspruch 5,
**dadurch gekennzeichnet,**
**dass** die einstellbaren Arretierungsmittel eine mit dem Vorrichtungsabschnitt (25) fest verbundene Zahnstange aufweisen und die Verschraubungsmittel (43) ein mit dem beweglichen Teil (29) fest verbundenes Rückschlag-Schnappsystem mit Betätigungsschlüssel aufweisen.

7. Vorrichtung nach Anspruch 6,
**dadurch gekennzeichnet,**
**dass** das Schnappsystem einen Hebelarm (50) zur Freigabe des Schnappers aufweist.

8. Vorrichtung nach einem der Ansprüche 5 bis 7,
**dadurch gekennzeichnet,**
**dass** die Verschraubungsmittel (43) mindestens eine Flügelschraube (49) aufweisen.

9. Vorrichtung nach einem der vorhergehenden Ansprüche,
**dadurch gekennzeichnet,**
**dass** sie ein Verbindungsteil (26) zwischen dem Stift (15) und dem Vorrichtungsabschnitt (25) umfasst, welches durch einen Block mit zwei parallel zueinander verlaufenden Schaftritzeln (27, 28) für das Winkelgetriebe für das Band (3) in dessen Ebene gebildet ist, wobei der Block mit dem Stift (15) fest verbunden ist.

10. Vorrichtung nach Anspruch 9,
**dadurch gekennzeichnet,**
**dass** das Verbindungsteil (26) entlang des Stiftes (15) beweglich ist.

11. Vorrichtung nach einem der vorhergehenden Ansprüche,
**dadurch gekennzeichnet,**
**dass** der Winkel des Vorrichtungsabschnitts (25) zum Stift (15) einstellbar ist.

12. Vorrichtung nach einem der vorhergehenden Ansprüche,
**dadurch gekennzeichnet,**
**dass** der Winkel des Vorrichtungsabschnitts (25) zum Stift (15) zwischen 90° und 130° liegt.

13. Vorrichtung nach Anspruch 12,
**dadurch gekennzeichnet,**
**dass** der Winkel des Vorrichtungsabschnitts (25) zum Stift (15) 90° beträgt.

14. Anordnung, **dadurch gekennzeichnet,**
**dass** sie eine Vorrichtung (14) nach einem der vorhergehenden Ansprüche und ein flexibles Band (3) umfasst.

## Claims

1. Device for tensioning a flexible band (3) for holding a bony element on an implant, including a rod (15) having a first end (16) provided with means (17) for bearing on a longitudinal bar for fixing the implant, a movable part (29) for attaching the flexible band (3) to a portion (25) of the device and means (42) for adjustably locking the movable part (29) in translation in relation to said portion (25) of the device, **characterised in that** the rod (15) comprises a gripping sleeve (23) at its second end (24), **in that** said portion (25) of the device (14) forms an angle with the rod (15) to which it is fixed rigidly at an intermediate level with angle guides (27, 28) for the band (3), and **in that**, said flexible band comprising two free ends (36, 37), said movable part (29) comprises a wheel (31) which is free to rotate in relation to an axis (32) perpendicular to said portion (25), for guiding the band (3) around its rounded circumference (33) including two separate elements (34, 35) for anchoring the flexible band (3), namely a first element (34) for one of said ends of the flexible band (3) and a second element (35) for the other end.

2. Device according to claim 1, **characterised in that** the two anchoring elements (34, 35) are substantially diametrically opposed.

3. Device according to claim 2, **characterised in that** the wheel (31) is in the form of a truncated circular or oval disk including a groove (38) for guiding the band (3) on its circumference (33).

4. Device according to any one of the preceding claims, **characterised in that**, the band (3) including on one side (36) an end piece in the form of a flexible strip and on the other side (37) an attachment pin (52), the first element (34) is formed by an oblique groove (40) in the plane of the wheel (31) for retention of the strip, and the second element (35) by a hook (41, 41') for attachment of the pin (52).

5. Device according to any one of the preceding claims, **characterised in that** the adjustable locking means (42) comprise screwing means (43) designed to operate the movable part (29).

6. Device according to claim 5, **characterised in that** the adjustable locking means (42) include a rack which is integral with the portion (25) of the device, and the screwing means (43) comprise a ratchet system with a non-return pawl and operating key integral with the movable part (29).

7. Device according to claim 6, **characterised in that** the ratchet system includes a lever arm (50) for releasing the pawl.

8. Device according to any one of claims 5 to 7, **characterised in that** the screwing means (43) includes at least one butterfly screw (49).

9. Device according to any one of the preceding claims, **characterised in that** it has a linking part (26) between the rod (15) and the portion (25) of the device formed by a block provided with two pinions (27, 28) with parallel axes forming angle guides for said band (3) in its plane, said block being integral with said rod (15).

10. Device according to claim 9, **characterised in that** the linking part (26) is movable along the rod (15).

11. Device according to any one of the preceding claims, **characterised in that** the angle of said portion (25) of the device in relation to the rod (15) is adjustable.

12. Device according to any one of the preceding claims, **characterised in that** the angle of said portion (25) of the device in relation to the rod (15) is between 90° and 130°.

13. Device according to claim 12, **characterised in that** the angle of said portion (25) of the device in relation to the rod (15) is 90°.

14. Set, **characterised in that** it comprises a device (14) according to any one of the preceding claims and a flexible band (3).
